# EUROPÄISCHE PATENTSCHRIFT

(11) **EP 1 154 812 B1**
(45) Veröffentlichungstag und Bekanntmachung des Hinweises auf die Patenterteilung: **28.05.2003**
(21) Anmeldenummer: 00908812.1
(22) Anmeldetag: 22.02.2000
(51) Int. Cl.: A61M 5/31, A61M 5/28

(54) **SPRITZENEINRICHTUNG**
SYRINGE
DISPOSITIF D'INJECTION

(30) Priorität: 23.02.1999 AT 11799
(43) Veröffentlichungstag der Anmeldung: 21.11.2001
(73) Patentinhaber: Baxter Vaccine Aktiengesellschaft, 1221 Wien (AT)
(72) Erfinder: MOSER, Franz, A-2232 Deutsch Wagram (AT); BUSCH-PETERSEN, Erik, A-1220 Wien (AT)
(74) Vertreter: Weinzinger, Arnulf, Dipl.-Ing.
(86) Internationale Anmeldenummer: AT0000045
(87) Internationale Veröffentlichungsnummer: WO00050108

(56) Entgegenhaltungen:
- EP-A- 0 685 237
- EP-A- 0 707 859
- EP-A- 0 716 860
- WO-A-94/22511
- US-A- 5 135 496
- US-A- 5 785 691

## Beschreibung

Die Erfindung betrifft eine Spritzeneinrichtung, gemäß dem einleitenden Teil von Anspruch 1.

Weiters bezieht sich die Erfindung auf eine Verschlusskappen-Einheit gemäß dem einleitenden Teil von Anspruch 19.

Im Zusammenhang mit Injektionsspritzen ist es vielfach bekannt geworden, die bereits mit dem Spritzenkörper verbundene Kanüle vor dem Gebrauch der Injektionsspritze mit einem speziellen Kanülenschutz zu umgeben und zu sichern. Dabei sind insbesondere Kanülenschutz-Ausbildungen vorgeschlagen worden, bei denen ein vorderer Schutzhüllenteil über eine Sollbruchstelle mit einem hinteren Befestigungsteil verbunden ist, vgl. beispielsweise die WO 94/2251 A, aber auch die AT 242 286 B. Bekannt ist es hierbei weiters, im Inneren des Kanülenschutzes einen Dichtkörper aus Gummi anzubringen, in den die Kanülenspitze zwecks Abdichtung einsticht, vgl. außer der bereits vorstehend genannten WO 94/22511 A auch beispielsweise die DE 848 081 C oder aber die US 4 735 311 A. Bei allen diesen bekannten Systemen handelt es sich jedoch um Schutzhüllen für Kanülen, die an Injektionsspritzenkörpern angebracht sind, wobei dadurch einerseits die Kanülen vor Umgebungseinflüssen geschützt und insbesondere steril gehalten werden sollen, andererseits Personen, die mit den Spritzen hantieren, vor Verletzungen geschützt werden sollen, bis die Injektionsspritzen benützt werden.

In der Praxis hat sich jedoch ergeben, dass Ärzte bei der Benützung von Injektionsspritzen je nach Patienten unterschiedliche Kanülen verwenden wollen, wobei der Arzt beispielsweise für Kinder dünnere, kürzere, für Erwachsene jedoch stärkere, längere Kanülen zu verwenden wünscht; die Wahl der Kanüle kann auch von Art und Ort der Injektion abhängen. Es werden daher Kanüleneinheiten, mit einem auf den üblicherweise konischen Anschlussabschnitt des Spritzenzylinders aufsetzbaren Anschlussteil, gesondert von den gefüllten und dicht verschlossenen Spritzenkörpern zur Verfügung gestellt, und bei der Verabreichung einer Injektion wählt dann der Arzt die jeweilige Kanüleneinheit aus einem Sortiment aus. Die Spritzenkörper werden demgemäß bis zu diesem Zeitpunkt durch eine einfache haubenförmige Verschlusskappe verschlossen, welche auf den Anschlusskonus des Spritzenkörpers aufgeschoben wird. Diese haubenförmigen Verschlusskappen sind jedoch mühsam in der Handhabung und unzuverlässig hinsichtlich eines dichten Verschlusses der Spritzenkörper. Ferner sind, wenn die Spritze benützt werden soll, die Verschlusskappen, sofern sie einen festen, dichten Sitz auf dem Anschlusskonus des Spritzenkörpers haben, nur mühsam zu entfernen, wobei dies zur Folge haben kann, dass beim Abnehmen dieser Verschlusskappen ein Teil der Injektionsflüssigkeit ungewollt verschüttet wird. Überdies erfordern diese Ausbildungen eine Umstellung vor allem im Zuge einer automatisierten Füllung und Assemblierung der Injektionsspritzen, bei der in herkömmlicher Weise bereits mit Kanülen versehene Spritzenkörper mit abdichtenden Kanülenschutzhüllen versehen, sodann gefüllt und schließlich an der Rückseite mit Hilfe von Kolben dicht verschlossen werden. Wenn dagegen die Spritzenkörper mit einer haubenförmigen Verschlusskappe verschlossen werden, müssen Greifelemente oder Halterungen in der bei der automatischen Befüllung und Assemblierung verwendeten Einrichtung anders gestaltet werden als bei Spritzenkörpern mit Kanüle und Kanülenschutz.

Dieser Nachteil trifft auch auf die Spritzeneinrichtung gemäß US 5 135 496 A zu, bei der ebenfalls eine mehr oder weniger haubenförmige Verschlusskappe vorliegt, die zwei über eine Sollbruchstelle verbundene Kappenabschnitte, einen hinteren Befestigungsabschnitt und einen vorderen Verschlussabschnitt aufweist, um so mehr Sicherheit bezüglich eines unbefugten Hantierens und eine Überprüfungsmöglichkeit hinsichtlich Sterilzustand zu erlangen. Jedoch ist hier ein gesonderter, abgestufter, hülsenförmiger Kanülen-Anschlussteil vorgesehen, der über einen weichen, elastomeren Stopfen mit mittiger Bohrung am Spritzenkörper angebracht ist; der Spritzenkörper selbst weist einen einfachen Hals mit verdicktem Bund auf, über den der Kanülen-Anschlussteil unter Aufschnappen aufgeschoben ist. Am vorderen Ende steht der Kanülen-Anschlussteil über dem weichen Stopfen stirnseitig vor, und in den so gebildeten Aufnahmeraum ragt ein Dichtstopfen, der mit einem mit ihm einstückigen, ihn außen umschließenden Schürzenteil auf den vorderen Bereich des Kanülen-Anschlussteils aufgesetzt ist. Wie ersichtlich ist diese Ausbildung ziemlich aufwendig und dennoch relativ unsicher, da sich der Dichtstopfen in seinem Sitz beim Hantieren mit der Spritzeneinrichtung lockern könnte.

Eine ähnliche Ausbildung, die als Ausgangspunkt für die vorliegende Erfindung anzusehen ist, ist aus der EP 707 859 A bekannt. Allerdings ist hier der Kanülen-Anschlussteil in herkömmlicher Weise durch einen Luer-Konusteil am Spritzenkörper selbst gebildet. Dieser Luer-Konusteil wird durch einen haubenförmigen Dichtkörper abgedichtet, der-an ihm direkt anliegt, und über dem eine Verschlusskappe angebracht ist, die in einer Ausführungsform aus zwei über eine Sollbruchstelle miteinander verbundenen Kappenabschnitten besteht.

Es ist nun Aufgabe der Erfindung, eine Spritzeneinrichtung bzw. eine Verschlusskappen-Einheit wie eingangs angeführt vorzusehen, die bei einer einfachen Ausbildung sowohl einen zuverlässigen dichten Verschluss gewährleisten als auch eine einfache Handhabung und Manipulation einerseits während des Herstellens bzw. Befüllens der Spritzenkörper sowie andererseits beim Vorbereiten der Spritzeneinrichtung, zum Anbringen der jeweiligen Kanüle, sicherstellen.

Zur Lösung dieser Aufgabe sieht die Erfindung eine Spritzeneinrichtung mit den Merkmalen des Anspruches 1 bzw. eine Verschlusskappen-Einheit mit den Merkmalen des Anspruches 19 vor. Vorteilhafte Ausführungsformen und Weiterbildungen sind in den Unteransprüchen definiert.

Mit einer erfindungsgemäßen Ausbildung ist eine bequeme Handhabung ebenso wie ein außerordentlich einfacher, nichtsdestoweniger sicherer, dichter Originalitätsverschluss des Spritzenkörpers möglich, und die Verschlusskappe ist auch in Verbindung mit einer automatischen Assemblierung und Befüllung der Spritzeneinrichtung gut geeignet, ohne dass Änderungen an bestehenden Anlagen notwendig wären, wie sie für Spritzen mit Kanülen verwendet werden. Zur Benützung der Spritzeneinrichtung wird, um eine Kanüle aufzusetzen, der vordere Verschlussabschnitt vom hinteren Befestigungsabschnitt an der Sollbruchstelle abgetrennt, und der Befestigungsabschnitt verbleibt am Spritzenkörper. Dabei kann der Befestigungsabschnitt einen Anschlussbereich für die Kanüle aufweisen, vorzugsweise wird jedoch, wie nachstehend dargelegt wird, ein mit dem Spritzenkörper einteiliger Kanülen-Anschlussteil oder ein gesonderter Kanülen-Anschlussteil eingesetzt.

Für die gute Positionierung und sichere Halterung des Dichtkörpers im Verschlussabschnitt ist es günstig, dass der Aufnahmeraum für den Dichtkörper an der vom Befestigungsabschnitt abgewandten vorderen Seite durch eine radiale Schulter begrenzt ist.

Wie vorstehend bereits angedeutet wird bei Verwendung der Spritzeneinrichtung die jeweilige Kanüleneinheit, nachdem der Verschlussabschnitt der Verschlusskappe an der Sollbruchstelle abgetrennt und entfernt worden ist, aufgesetzt, wobei dann nicht unbedingt ein Kanülen-Anschlussteil des Spritzenkörpers verwendet werden muss, sondern ein gesonderter Anschlussteil in der Verschlusskappe eingesetzt werden kann. Hierfür hat sich eine Ausführungsform als besonders vorteilhaft erwiesen, bei der im Übergangsbereich von Befestigungsabschnitt und Verschlussabschnitt, beidseits der Sollbruchstelle, innen ein gesonderter, mit einer axialen Bohrung versehener Kanülen-Anschlussteil aufgenommen ist, dessen Sitz im Befestigungsabschnitt fester als im Verschlussabschnitt ist. Bei dieser Ausführungsform liegt der gesonderte Kanülen-Anschlussteil geschützt im Inneren der Verschlusskappe vor, so dass er während des Transports und der Lagerung im Inneren steril gehalten werden kann. Der gesonderte Kanülen-Anschlussteil ermöglicht auch eine einfache Fertigung der Verschlusskappe, in die er nach deren Fertigung, von der Rückseite her, eingesetzt wird, nachdem zuvor der Dichtkörper eingesetzt wurde. Bei auf den Spritzenkörper aufgesetzter Verschlusskappe verlängert dieser Kanülen-Anschlussteil in der Verschlusskappe de facto den Kanülen-Anschlussteil des Spritzenkörpers. Damit dieser gesonderte Kanülen-Anschlussteil beim Abtrennen des Verschlussabschnittes der Verschlusskappe nicht ungewollt mit entfernt wird, ist sein Sitz im Befestigungsabschnitt der Verschlusskappe fester als jener im Verschlussabschnitt. Dies ergibt sich im Übrigen mehr oder weniger von selbst, wenn der gesonderte Kanülen-Anschlussteil mit seinem vorderen, im Verschlussabschnitt befindlichen Bereich als Luer-Konus ausgebildet ist. Durch die konische Ausbildung des vorderen Bereichs des gesonderten Kanülen-Anschlussteils ist der Halt dieses Bereichs im vorderen Verschlussabschnitt der Verschlusskappe nur gering, wobei die Innenwandung des Verschlussabschnitts der Verschlusskappe über den größten Teil nicht oder nur lose am gesonderten Kanülen-Anschlussteil anliegt. Festgehalten wird dieser gesonderte Kanülen-Anschlussteil demgemäß im Befestigungsabschnitt, u.zw. vorzugsweise in einer Position, in der er, wenn die Verschlusskappe auf dem Spritzenkörper angebracht ist, mit der hinteren Stirnseite an der vorderen Stirnseite des Kanülen-Anschlussteils des Spritzenkörpers anliegt.

Es hat sich hier auch als vorteilhaft erwiesen, wenn der gesonderte Kanülen-Anschlussteil in seinem hinteren Bereich außen mit einer Umfangsrille und/oder einem Umfangswulst zur Verstärkung des Sitzes im Befestigungsabschnitt geformt ist. Durch diese Elemente für einen gegenseitigen Eingriff von Befestigungsabschnitt und Kanülen-Anschlussteil zur Erzielung einer Art formschlüssigen Verbindung wird überdies eine gute Positionierung des gesonderten Kanülen-Anschlussteils im Inneren der Verschlusskappe sichergestellt.

In diesem Zusammenhang ist es weiters günstig, wenn im Verschlussabschnitt ein radialer Absatz geformt ist, der die Position des gesonderten Kanülen-Anschlussteils nach vorne begrenzt.

Der gesonderte Kanülen-Anschlussteil kann problemlos aus einem anderen Material, insbesondere einem härteren Material, als die Verschlusskappe gefertigt werden, was für die Anbringung der Kanüleneinheit von Vorteil ist, und insbesondere hat sich bei Versuchen Polycarbonat, nämlich Makrolon, als vorteilhaft erwiesen.

Üblicherweise ist der Spritzenkörper mit einem Kanülen-Anschlussteil in Form eines Luer-Konus ausgeführt, und dementsprechend ist bei der vorliegenden Verschlusskappe in vorteilhafter Weise der Befestigungsabschnitt in an sich bekannter Weise für den Sitz auf dem konischen Kanülen-Anschlussteil des Spritzenkörpers mit einem Innenkonus ausgebildet.

Wenn andererseits eine besonders einfache Ausbildung angestrebt wird, kann mit Vorteil vorgesehen sein, dass der Befestigungsabschnitt der Verschlusskappe ringförmig ausgebildet ist. Dabei ist es weiters im Hinblick auf eine minimale Anzahl von Komponenten günstig, wenn der Kanülen-Anschlussteil durch einen vorderen Anschluss-Abschnitt des Spritzenkörpers selbst gebildet ist, an dessen hinterem Ende der ringförmige Befestigungsabschnitt gehalten ist. Bei dieser Ausbildung wird zweckmäßig, um die Manipulationen an gängigen Maschinen beim Assemblieren und Füllen der Spritzeneinrichtung zu ermöglichen, der vordere Verschlussabschnitt der Verschlusskappe entsprechend (besonders) lang gestaltet (nämlich in Entsprechung zu einer Kanülen-Schutzkappe, wenn eine Kanüle vorhanden wäre), was ohne Beeinträchtigung der Dichtwirkung durch den Stopfen (und ohne eine überlange Ausbildung desselben) möglich ist, da der Stopfen nach vorne durch die radiale Schulter gesichert ist.

Der Kanülen-Anschlussteil begrenzt mit seiner vorderen Stirnseite den Aufnahmeraum für den Dichtkörper. Dabei ist es weiters für eine gute Abdichtung der axialen Bohrung des Kanülen-Anschlussteils vorteilhaft, wenn der Dichtkörper, d.h. Stopfen unter axialer Kompression zwischen der vorderen Stirnseite des Kanülen-Anschlussteils und der radialen Schulter im Verschlussabschnitt gehalten ist.

Für die einfache Handhabung ist es auch von Vorteil, wenn der Verschlussabschnitt und/oder der Befestigungsabschnitt an der Außenseite in an sich bekannter Weise mit Griffvorsprüngen, z.B. Längsrippen, ausgebildet ist bzw. sind. Dadurch können diese Abschnitte der Verschlusskappe beim Abtrennen des Verschlussabschnitts sicher ergriffen und festgehalten werden, und der Verschlussabschnitt kann bequem in einer Dreh-Zug-Bewegung entfernt werden.

Um die Abnahme des Verschlussabschnittes zu erleichtern, ohne dass der Befestigungsabschnitt dabei ungewollt vom Spritzenkörper getrennt wird, ist es auch günstig, wenn der Befestigungsabschnitt, z.B. an seinem hinteren, vom Verschlussabschnitt abgewandten Ende, in an sich bekannter Weise mit Schnappverbindungsteilen für einen Schnappsitz auf einem Kanülen-Anschlussteil des Spritzenkörpers geformt ist. Die Schnappverbindungsteile können dabei ähnlich wie etwa in der WO 94/22 511 A im Zusammenhang mit einem Kanülenschutz beschrieben ausgebildet sein.

Um den in den Aufnahmeraum eingesetzten Dichtkörper einerseits durch örtliche radiale Kompression festzuhalten und andererseits während des Einsetzens des Dichtkörpers in den Verschlussabschnitt, der an der vorderen Stirnseite verschlossen ist, ein Entweichen der vor dem Dichtkörper befindlichen Luft zu ermöglichen, hat es sich auch als vorteilhaft erwiesen, wenn der Dichtkörper-Aufnahmeraum im Verschlussabschnitt mit Längsstegen und dazwischenliegenden Lüftungskanälen ausgebildet ist.

Als Material für die Verschlusskappe, d.h. für deren Befestigungs- und Verschlussabschnitt, wird zweckmäßigerweise ein nicht zu starres, eher weicheres Kunststoffmaterial verwendet, welches im Übrigen für medizinische Zwecke geeignet sein sollte. Vorzugsweise wird hierfür Polyethylen verwendet, wobei die beiden Kappenabschnitte aus dem Polyethylen durch Spritzgießen geformt werden können. Mit einem derartigen Material wird nicht nur eine einfache preiswerte Massenfertigung ermöglicht, sondern auch ein dichtes Aufsetzen und Verbinden, insbesondere am Spritzenkörper, sichergestellt, wobei sich das Verschlusskappen-Material etwas zusammendrücken kann. Dies ist insbesondere dann zweckmäßig, wenn wie vorstehend erwähnt eine Schnappverbindung zusätzlich zur Anwendung kommt.

Die Verschlusskappe wird als einstückiges Element zur Verfügung gestellt, und als solches kann sie auch vorgefertigt werden. Für die Anbringung am Spritzenkörper wird dann der gesonderte Dichtkörper eingesetzt. Der Dichtkörper besteht einfach aus einem zylindrischen Stopfen, der insbesondere aus Latex-freiem Gummi gefertigt ist. Ein derartiges Material ist für medizinische Anwendungen geeignet und kann mit vielen gängigen Injektionsflüssigkeiten, wie etwa solchen für eine FSME-Schutzimpfung, in Kontakt gelangen.

Vorzugsweise ist der Spritzenkörper bereits mit einer Injektionsflüssigkeit gefüllt und an seinem von der Verschlusskappe abgewandten hinteren Ende mit einem Kolben verschlossen.

Der als gesonderte Komponente gefertigte Kanülen-Anschlussteil kann zusammen mit dem als Dichtkörper vorgesehenen Stopfen in einer Verschlusskappe vormontiert werden, um eine als solche auf den Spritzenkörper zu montierende Verschlusskappen-Einheit, wie in Anspruch 19 definiert, zu bilden.

Die Erfindung wird nachstehend anhand von bevorzugten Ausführungsbeispielen, auf die sie jedoch nicht beschränkt sein soll, und unter Bezugnahme auf die Zeichnung noch weiter erläutert. Es zeigen:
Fig.1 eine teilweise aufgebrochene Ansicht einer Ausführungsform einer Spritzeneinrichtung ohne Kanüleneinheit, jedoch mit Verschlusskappe;
Fig.2 den vorderen Bereich dieser Spritzeneinrichtung, mit einer Längsschnittdarstellung der auf den Spritzenkörper aufgesetzten Verschlusskappe;
Fig.3 einen Querschnitt durch die Verschlusskappe gemäß der Linie III-III in Fig.2;
Fig.4 in einem Längsschnitt die Verschlusskappe ohne Kanülen-Anschlussteil und ohne Dichtkörper;
Fig.5 in einem Axialschnitt den Kanülen-Anschlussteil für die Verschlusskappe gemäß Fig.4;
die Fig.6 und 7 in schematischen Darstellungen, teilweise im Längsschnitt, den Verschlusskappenbereich der Injektionsspritze, wobei in Fig.6 die Abnahme des vorderen Verschlussabschnittes der Verschlusskappe zwecks Inbetriebnahme der Injektionsspritze und in Fig.7 das Aufsetzen einer Kanüleneinheit auf den Kanülen-Anschlussabschnitt der Verschlusskappe veranschaulicht ist;
Fig.8 eine Seitenansicht des vorderen Bereichs einer anderen Ausführungsform einer Spritzeneinrichtung, mit einem teilweise dargestellten Spritzenkörper und einer aufgesetzten Verschlusskappe;
Fig.9 diese Spritzeneinrichtung in einer Längsschnittdarstellung der Verschlusskappe, ähnlich der Darstellung in Fig.2;
Fig. 10 einen Querschnitt durch die Verschlusskappe gemäß der Linie X-X in Fig.9;
Fig.11 eine mit Fig.9 vergleichbare Darstellung des vorderden Bereichs der Spritzeneinrichtung, wobei jedoch der vordere Verschlussabschnitt der Verschlusskappe bereits abgetrennt veranschaulicht ist; und
Fig.12 den vorderen Teil des Spritzenkörpers mit dem auf seinem Kanülen-Anschlussteil verbliebenen Befestigungsabschnitt der Verschlusskappe, wobei das Aufsetzen einer Kanülen-Einheit veranschaulicht ist.

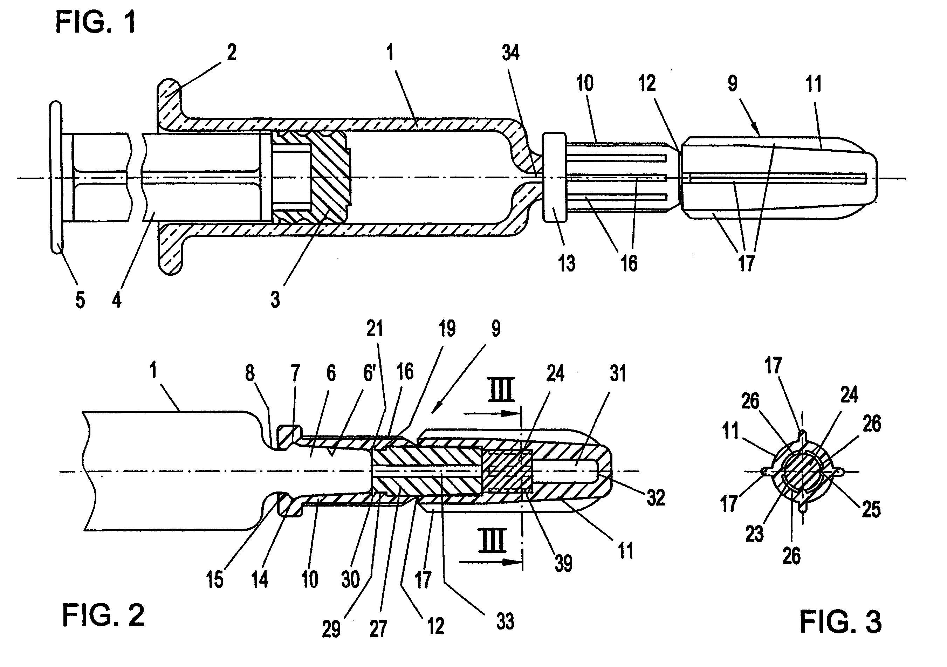

In den Fig.1 bis 3 ist eine Spritzeneinrichtung ohne Kanüle veranschaulicht, die einen üblicherweise aus Glas oder transparentem Kunststoff bestehenden Spritzenkörper 1 aufweist, der an seinem hinteren, offenen, mit einem Fingergriff 2 versehenen Ende durch einen Kolben 3 dicht verschlossen wird; mit diesem Kolben 3 ist eine Kolbenstange 4 mit einem Griff 5 verbunden.

Am gegenüberliegenden, vorderen Ende weist der Spritzenkörper 1 einen üblichen einteiligen, konischen Kanülen-Anschlussteil 6 auf, der über einen Umfangswulst 7 und einen vergleichsweise engen Halsabschnitt 8 in den Zylinder des Spritzenkörpers 1 übergeht. Auf diesen Kanülen-Anschlussteil 6 ist gemäß Fig.1 und 2 eine Verschlusskappe 9 aufgesetzt, die zwei längliche, einteilig miteinander verbundene Kappenabschnitte, nämlich einen hinteren Befestigungsabschnitt 10 und einen vorderen, zur Abdichtung dienenden Verschlussabschnitt 11 enthält. Diese beiden Kappenabschnitte 10, 11 sind durch eine Sollbruchstelle 12, die durch eine Materialverengung gebildet ist, einstückig miteinander verbunden.

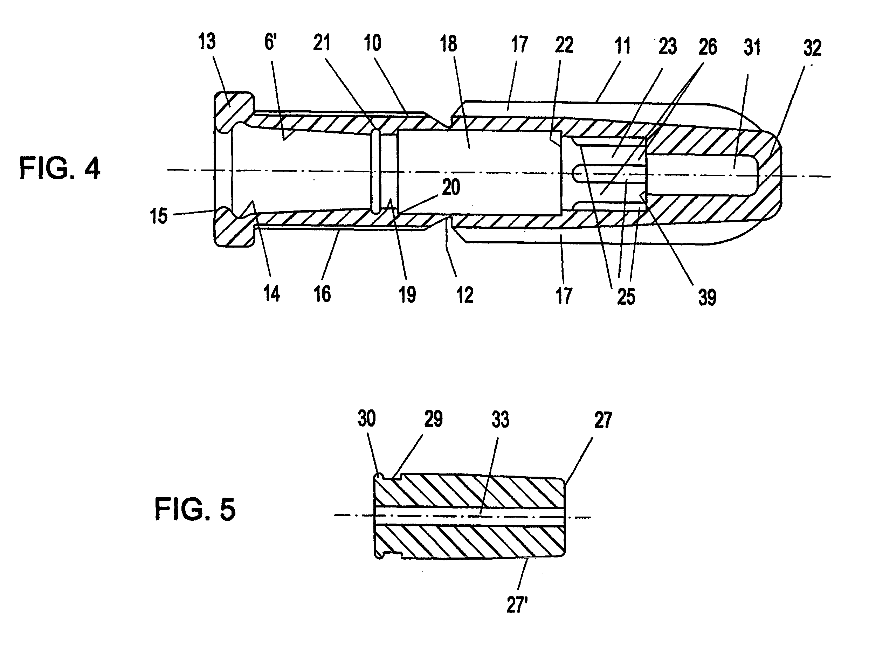

Der Befestigungsabschnitt 10 weist an seinem hinteren Ende einen mit ihm einstückig geformten Bund 13 auf, und seine Innenwandung 6' ist - zumindest im hinteren Bereich, vgl. außer Fig.2 und Fig.4 - entsprechend dem konischen Anschlussteil 6 des Spritzenkörpers 1 konisch sowie im Bereich des Bundes 13 mit einer zum Umfangswulst 7 bzw. zur Halsverengung 8 passenden Umfangsnut 14 bzw. einem gegenüber dieser Umfangsnut 14 radial einwärts vorspringenden Umfangswulst 15 versehen. An seiner Außenseite ist der Befestigungsabschnitt 10 mit mehreren sich in Längsrichtung erstreckenden Griffvorsprüngen in Form von Längsrippen 16 geformt. Die Umfangsnut 14 und der Umfangswulst 15 bilden Schnappverbindungsteile zum festen Anbringen des Befestigungsabschnittes 10 und damit der Verschlusskappe 9 am Kanülen-Anschlussabschnitt 6 des Spritzenkörpers 1, wie in Fig.2 veranschaulicht ist.

Der Verschlussabschnitt 11 der Verschlusskappe 9 weist ebenfalls an seiner Außenseite Längsrippen 17 als Griffvorsprünge auf, und er ist in seinem hinteren Bereich mit einem zylindrischen Innenraum 18 ausgebildet, welcher sich über den Bereich der Sollbruchstelle 12 hinweg bis in den vorderen Endbereich des Befestigungsabschnittes 10 der Verschlusskappe 9 erstreckt, s. insbesondere Fig.4. Hinter diesem zylindrischen Innenraum 18 schließt ein radial einwärts vorspringender Umfangswulst 19 über einen radialen Absatz 20 an, und dieser Ümfangswulst 19 geht danach in eine im Querschnitt kreisbogenförmige Umfangsnut 21 am hinteren Ende über.

Am vorderen Ende des zylindrischen Innenraums 18 geht dieser Innenraum 18 über einen allgemein rechtwinkeligen radialen Absatz 22 in einen Aufnahmeraum 23 für einen Dichtkörper 24 über. Dieser Aufnahmeraum 23 ist ebenfalls allgemein zylindrisch, wie sich außer aus Fig.4 auch aus Fig.3 entnehmen lässt, wobei jedoch der Aufnahmeraum 23 mit beispielsweise vier einwärts vorspringenden Längsstegen 25 geformt ist, die einerseits den Dichtkörper 24 (vgl. Fig.3) etwas eindrücken und andererseits dazwischenliegende Lüftungskanäle 26 definieren. Dadurch kann beim Einsetzen des zylindrischen Dichtkörpers 24 von der Rückseite (mit dem Bund 13) der Verschlusskappe 9 her (s. Fig.4) durch den Innenraum 18 in den Aufnahmeraum 23 die vor dem Dichtkörper 24 befindliche Luft während dessen Einschiebens in den Aufnahmeraum 23 durch die Lüftungskanäle 26 nach hinten entweichen. Die Längsstege 25 hingegen bewirken einen örtlichen Presssitz des Dichtungskörpers 24 im Aufnahmeraum 23, so dass dieser an einem ungewollten Herausfallen aus der Verschlusskappe 4 während des Zusammenbaus der Verschlusskappe 9 gehindert wird.

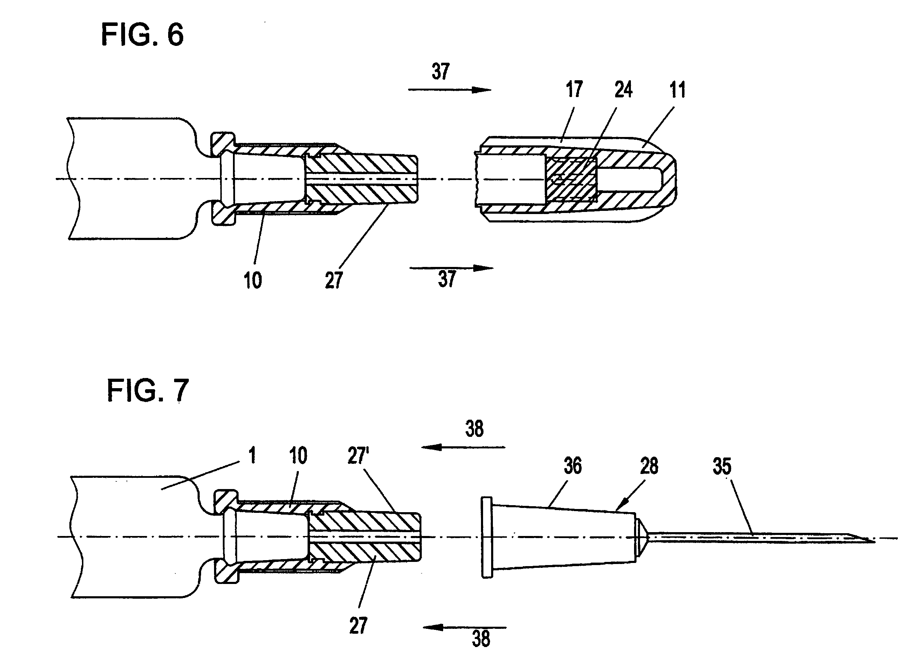

Hinter dem Dichtkörper 24 wird ein gesonderter Kanülen-Anschlussteil 27 im zylindrischen Innenraum 18 eingesetzt, um später, bei In-Betrieb-Nahme der Spritze, eine Kanüle anbringen zu können, wie nachstehend anhand der Fig.6 und 7 noch näher erläutert werden wird. Dieser gesonderte Kanülen-Anschlussteil 27 ist an seiner Außenseite über den größten Teil seiner Längserstreckung konisch geformt, um so einen üblichen Luer-Konus 27' für die aufzusetzende Kanüleneinheit 28 (Fig.7) vorzusehen. Dahinter ist eine zum Umfangswulst 19 passende Umfangsrille 29 mit anschließendem Umfangswulst 30 vorgesehen, wobei letzterer in die Umfangsnut 21 der Verschlusskappe 9 passt.

Die so mit Dichtkörper 24 und Anschlussteil 27 komplettierte Verschlusskappe 9 wird auf den Spritzenkörper 1 wie vorstehend anhand von Fig.2 erläutert aufgesetzt.

Der im vorderen Bereich des Verschlussabschnittes 11 der Verschlusskappe 9 vorgesehene axiale Hohlraum 31 ist aus herstellungstechnischen Gründen vorgesehen, wobei jedoch die Verschlusskappe 9 am vorderen Ende 32 abgeschlossen ist.

Der gesonderte Kanülen-Anschlussteil 27 weist gemäß Fig. 2 und 5 eine axiale Bohrung 33 auf, die mit der üblichen Bohrung 34 im Kanülen-Anschlussteil 6 des Spritzenkörpers 1 (s. Fig.1) fluchtet und somit in Strömungsverbindung steht. Dadurch kann Injektionsflüssigkeit aus dem Inneren des Spritzenkörpers 1 durch die axiale Bohrung 34 des Anschlussteils 6 des Spritzenkörpers 1 sowie die axiale Bohrung 33 des gesonderten Kanülen-Anschlussteiles 27 gedrückt werden, wenn der Kolben 3 nach vorne, d.h. im Spritzenkörper 1 einwärts (gemäß der Darstellung in Fig.1 nach rechts) bewegt wird.

Um die Spritzeneinrichtung gebrauchsfertig zu machen, muss am bisher beschriebenen Spritzenkörper 1 eine Kanüle angebracht werden, wobei dann eine Kanüleneinheit 28 nach Wunsch, mit kleinerer oder größerer, dünnerer oder dickerer Kanüle 35 (s. Fig.7) ausgewählt werden kann. Um eine solche Kanüle 35 bzw. eine Kanüleneinheit 28 (üblicherweise ist die Kanüle 35 mit einem konischen Kunststoff-Aufsetzteil 36 verklebt) aufsetzen zu können, muss ein entsprechender konischer Anschlussteil des Spritzenkörpers freigelegt werden. Im vorliegenden Fall wird für diese Zwecke der gesonderte Kanülen-Anschlussteil 27 mit seinem vorderen, konischen Bereich freigelegt, wozu der vordere Kappenabschnitt 11, nämlich der Verschlussabschnitt, an der Sollbruchstelle 12 abgetrennt wird, wie dies in Fig.6 schematisch veranschaulicht ist. Hiefür wird der vordere Verschlussabschnitt 11 gegenüber dem hinteren Befestigungsabschnitt 10 und dem Spritzenkörper 1 verdreht und sodann in Richtung der Pfeile 37 in Fig.6 abgezogen. Dabei ermöglichen die Längsrippen 17 einen festen Griff am Verschlussabschnitt 11. Beim Abtrennen des Verschlussabschnittes 11 wird zugleich auch der davor die axiale Bohrung 33 des Anschlussteils 27 dicht verschließende Dichtkörper 24 mit entfernt. Die Abnahme des Verschlussabschnitts 11 wird dabei auch insofern erleichtert, als dieser mit seiner zylindrischen Innenwandung (zylindrischer Innenraum 19) relativ lose über den konischen vorderen Abschnitt 27' des Kanülen-Anschlussteils 27 passt, s. auch die Darstellung in Fig.2, so dass nicht nur dem Abziehen des Verschlussabschnittes 11 nach Abdrehen der Sollbruchstelle 12 kaum ein Widerstand entgegengesetzt wird, sondern auch sichergestellt ist, dass der gesonderte Kanülen-Anschlussteil 27 am Befestigungsabschnitt 10 verbleibt, wo er über die formschlüssige Verbindung zufolge der Umfangsrillen oder -nuten 21 bzw. 29 und Umfangswulste 19 bzw. 30 einen vergleichsweise festen Sitz hat. Diese Rast- oder Formschluss-Verbindungselemente 19-29 bzw. 21-30 bewirken im Übrigen auch eine dichte Verbindung zwischen dem Befestigungsabschnitt 10 und dem gesonderten Kanülen-Anschlussteil 27, so dass zwischen diesen beiden Komponenten keine Injektionsflüssigkeit austreten kann.

Nach dem Abnehmen des vorderen Verschlussabschnittes 11 der Verschlusskappe 9 (Fig.6) wird die Kanüleneinheit 28 mit ihrem Kunststoffteil 36 auf den am Spritzenkörper 1 mittels des Befestigungsabschnitts 10 gehaltenen gesonderten Kanülen-Anschlussteil 27 aufgesetzt, vgl. Fig.7 und die Pfeile 38 in dieser Fig.7. Danach ist die Spritzeneinrichtung gebrauchsfertig, so dass die Injektion verabreicht werden kann.

Die Herstellung der Einheit gemäß Fig.1 und 2, also Spritzenkörper 1 mit Verschlusskappe 9 mit eingesetztem Dichtkörper 24 und gesondertem Kanülen-Anschlussteil 27, jedoch ohne Kanüle 35, kann derart erfolgen, dass die eigentliche Verschlusskappe 9, der Kanülen-Anschlussteil 27 und der Dichtkörper 24 ebenso wie der Spritzenkörper 1 unabhängig voneinander hergestellt werden. Die Verschlusskappe 9 wird beispielsweise aus einem relativ weichen Kunststoffmaterial, wie Polyethylen, im Spritzguss erzeugt. Der gesonderte Kanülen-Anschlussteil 27 wird aus einem härteren Kunststoffmaterial, wie dem unter der Bezeichnung Makrolon bekannten Polycarbonat, gefertigt, und der Dichtkörper 24 ist ein einfacher zylindrischer Stopfen, insbesondere aus einem Latex-freien Gummimaterial.

Diese Komponenten können dann automatisch montiert werden, indem zunächst der Dichtkörper 24 von der durch den Bund 13 definierten Rückseite der Verschlusskappe 9 her wie beschrieben in das Innere derselben eingeschoben wird, bis er an einer an den vorderen axialen Hohlraum 31 anschließenden radialen Schulter 39 am Übergang zum Durchmesser-größeren Aufnahmeraum 24 zur Anlage kommt. Danach wird der gesonderte Kanülen-Anschlussteil 27 ebenfalls von der Rückseite der Verschlusskappe 9 in diese eingesetzt, wobei der hintere Befestigungsabschnitt 10 durch den vorderen konischen Bereich 27' des gesonderten Kanülen-Anschlussteils 27 allmählich aufgeweitet wird und sodann mit seinem Umfangswulst 19 in die Umfangsrille 29 des Anschlussteils 27 einschnappt, wobei gleichzeitig der im Querschnitt gerundete Umfangswulst 30 des Anschlussteils 27 in die Umfangsnut 21 des Befestigungsabschnittes 10 einschnappt. Danach wird die so mit dem Dichtkörper 24 und dem Kanülen-Anschlussteil 27 versehene Verschlusskappe 9 strahlensterilisiert und schließlich auf den Anschlussabschnitt 6 des Spritzenkörpers 1 aufgesetzt, wobei die Schnappverbindungsteile 7, 8 bzw. 14, 15 eine feste Schnappverbindung ergeben. Dieses Montieren kann völlig automatisch erfolgen, wobei Sterilitätsprobleme vermieden werden können.

Nach dem Montieren der Verschlusskappe 9 am Spritzenkörper 1 wie vorstehend beschrieben wird der Spritzenkörper 1 von seiner Rückseite, wo die Fingergriffe 2 angebracht sind, mit der jeweiligen Injektionsflüssigkeit befüllt, und er wird danach mit dem Kolben 3 dicht verschlossen. An der Rückseite des Kolbens 3 wird sodann - bevorzugt ebenfalls automatisch - die Kolbenstange 4 angesetzt; die so erhaltene Spritzeneinrichtung ohne Kanüle ist dadurch dicht verschlossen, und sie wird über ein Transportsystem aus der Montage- und Füllstation heraustransportiert, in ein Tray eingelegt sowie zuletzt in einer Verpackung eingeschweißt.

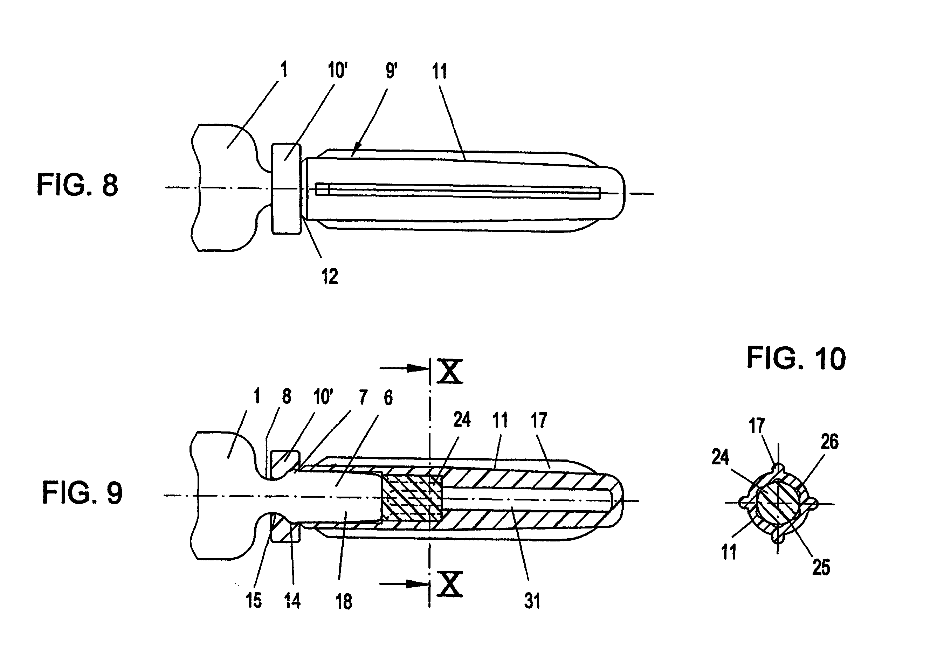

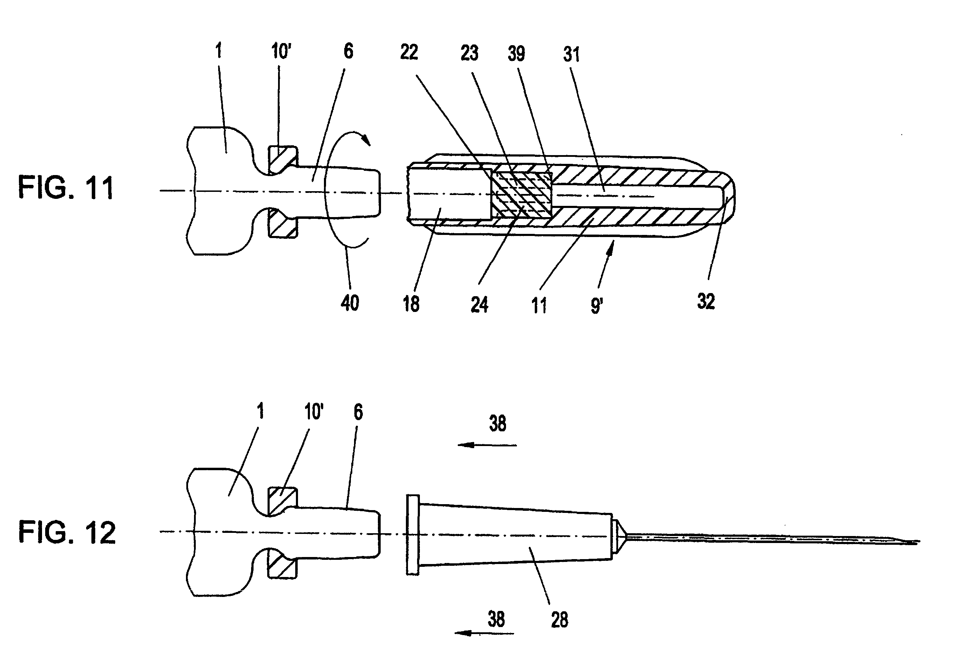

Bei der Ausführungsform gemäß Fig.8 bis 12 wird in Abweichung von der Ausführungsform gemäß Fig.1 bis 7 kein gesonderter Kanülen-Anschlussteil verwendet, vielmehr wird der mit dem Spritzenkörper 1 einteilige Kanülen-Anschlussteil 6 zur Befestigung der Kanüleneinheit 28 herangezogen, s. Fig.12. Dementsprechend fällt diese Ausführungsform zwar unter Anspruch 1, nicht jedoch unter Anspruch 19, und es liegt hier eine etwas modifizierte Verschlusskappe 9' vor, die zwar einen vorderen länglichen Verschlussabschnitt 11, aber anders als die Ausführungsform gemäß Fig.1 bis 7 einen ring- oder bundförmigen hinteren Befestigungsabschnitt 10' aufweist. Die beiden Kappenabschnitte 10',11 sind wiederum über eine Sollbruchstelle 12 miteinander einstückig verbunden. Wie aus Fig.9 ersichtlich ist der ringförmige Befestigungsabschnitt 10' in ähnlicher Weise wie der Befestigungsabschnitt 10 in der Ausführungsform gemäß Fig.2 mit Hilfe eines Umfangswulstes 15 und einer Umfangsnut 14 auf einer Halsverengung 8 bzw. auf einem Umfangswulst 7 am hinteren Ende des Kanülen-Anschlussteiles 6 des Spritzenkörpers 1, an dessen Übergang zum Spritzenzylinder des Spritzenkörpers 1, durch Aufschnappen befestigt. Insofern liegt eine zur Ausführungsform gemäß Fig.2 gleiche Ausbildung der Schnappverbindungsteile vor.

Der vordere Verschlussabschnitt 11 der Verschlusskappe 9' ist grundsätzlich gleich wie der Verschlussabschnitt 11 in der Ausführungsform gemäß Fig.2 ausgebildet, d.h. er hat in ähnlicher Weise außen Längsrippen 17 zur Erleichterung des festen Ergreifens beim Abtrennen des Verschlussabschnittes 11 (vgl. Fig.11), und weiters schließt über einen radialen Absatz 22, der als Anschlag für die Stirnseite des Kanülen-Anschlussteils 6 des Spritzenkörpers 1 dient, ein Aufnahmeraum 23 für einen als Dichtkörper vorgesehenen zylindrischen Stopfen 24 an, vgl. insbesondere Fig.11. Am vorderen Ende liegt der Stopfen 24 an einer den Aufnahmeraum 23 nach vorne abschließenden radialen Schulter 39 an, die einen Übergang zu einem vorderen, Durchmesser-kleineren axialen Hohlraum 31 bildet. Dieser Hohlraum 31 ist durch die stirnseitige Kappenwand 32 der Verschlusskappe 9' abgeschlossen.

Hinter dem Dichtstopfen 24 bzw. hinter dem radialen Absatz 22 befindet sich im Verschlussabschnitt 11 der Verschlusskappe 9' wiederum ein zylindrischer oder aber gegebenenfalls leicht konischer Innenraum 18, in den in der montierten Stellung (s. Fig.9) der Kanülen-Anschlussteil 6 des Spritzenkörpers 1 hineinragt. Der Sitz des Kanülen-Anschlussteils 6 im Innenraum 18 ist dabei relativ lose, so dass die Abtrennung des vorderen Verschlussabschnittes 11 der Verschlusskappe 9' durch Drehen (s. Pfeil 40 in Fig.11) und Abziehen (entsprechend den Pfeilen 37 in Fig.6) problemlos und ohne Reibungswiderstand bewerkstelligt werden kann. Bei diesem Abtrennen des Verschlussabschnitts 11 der Verschlusskappe 9' verbleibt der Befestigungsabschnitt 10' als Bund oder Ring am hinteren Ende des Kanülen-Anschlussteils 6 des Spritzenkörpers 1, ohne jedoch das nachfolgende Aufsetzen der Kanülen-Einheit 28 auf den Kanülen-Anschlussteil 6 des Spritzenkörpers 1 zu behindern; er kann gegebenenfalls beim Aufsetzen der Kanüleneinheit 28 einen hinteren Anschlag bilden. Das Aufsetzen der Kanüleneinheit 28 ist in Fig.12 wiederum mit Pfeilen 38 veranschaulicht.

In ähnlicher Weise wie vorstehend anhand der Fig.3 erläutert ist auch bei der Ausführungsform gemäß Fig.8 bis 12 eine Ausbildung des Aufnahmeraums 23 für den Dichtstopfen 24 mit Lüftungskanälen 26 und einen örtlichen Presssitz des Dichtungsstopfens 24 bewirkenden Längsstegen 25 vorgesehen, s. Fig.10.

Bei der Montage der Spritzeneinrichtung gemäß Fig.8 bis 12 kann ähnlich wie vorstehend anhand der Ausführungsform gemäß Fig.1 bis 7 erläutert vorgegangen werden, mit dem einzigen Unterschied, dass das Einsetzen des gesonderten Kanülen-Anschlussteils 27 entfällt. Es wird somit nach der gesonderten Fertigung der einzelnen Teile 1, 9' und 24 der Dichtstopfen 24 von hinten in die Verschlusskappe 9' eingesetzt, und danach wird die Verschlusskappe 9' auf den Kanülen-Anschlussteil 6 des Spritzenkörpers 1 aufgeschnappt. Im Anschluss daran wird wiederum der Spritzenkörper 1 von der in Fig.8 bis 12 nicht näher gezeigten Rückseite her befüllt und rückseitig mit einem Kolben (3 in Fig.1) verschlossen.

Auch in der Ausführungsform gemäß Fig.8 bis 12 kann die Verschlusskappe 9' aus einem relativ weichen Kunststoffmaterial, wie Polyethylen, bestehen und durch Spritzgießen erzeugt werden. Der Dichtstopfen 24 besteht wiederum bevorzugt aus einem Latex-freien Gummimaterial, so dass er in der montierten Stellung gemäß Fig.9 in axialer Richtung komprimiert werden kann, um die Dichtungswirkung auch bei Fertigungstoleranzen immer sicherzustellen und gegebenenfalls zu verstärken.

## Patentansprüche

1. Spritzeneinrichtung mit einem Spritzenkörper (1), an dessen einem, vorderen Ende ein Kanülen-Anschlussteil (6;27) mit einer axialen Bohrung (33,34) für das Aufsetzen einer Kanüleneinheit (28) vorgesehen ist, mit einem den Kanülen-Anschlussteil abdichtenden Dichtkörper (24) und einer Verschlusskappe (9,9') zum dichten Verschließen des Kanülen-Anschlussteils vor der Anbringung der Kanüleneinheit (28), wobei die Verschlusskappe zwei über eine Sollbruchstelle (12) einteilig miteinander verbundene Kappenabschnitte (10, 11) aufweist, von denen der eine ein am Kanülen-Anschlussteil angebrachter hinterer Befestigungsabschnitt (10,10') und der andere ein einen Aufnahmeraum (23) für den Dichtkörper (24) aufweisender vorderer Verschlussabschnitt (11) ist, **dadurch gekennzeichnet, dass** der Dichtkörper (24) ein zylindrischer Stopfen (24) ist, der zwischen der vorderen Stirnseite des Kanülen-Anschlussteils (6;27) und einer den Aufnahmeraum (23) vorderseitig begrenzenden radialen Schulter (39) im Inneren des länglichen Verschlussabschnitts (11) der Verschlusskappe (9,9') eingelegt ist.

2. Spritzeneinrichtung nach Anspruch 1, **dadurch gekennzeichnet, dass** im Übergangsbereich von Befestigungsabschnitt (10) und Verschlussabschnitt (11), beidseits der Sollbruchstelle (12), innen ein gesonderter, mit einer axialen Bohrung versehener Kanülen-Anschlussteil (27) aufgenommen ist, dessen Sitz im länglichen Befestigungsabschnitt (10) fester als im Verschlussabschnitt (11) ist.

3. Spritzeneinrichtung nach Anspruch 2, **dadurch gekennzeichnet, dass** der gesonderte Kanülen-Anschlussteil (27) mit seinem vorderen, im Verschlussabschnitt (11) befindlichen Bereich als Luer-Konus ausgebildet ist.

4. Spritzeneinrichtung nach Anspruch 2 oder 3, **dadurch gekennzeichnet, dass** der gesonderte Kanülen-Anschlussteil (27) in seinem hinteren Bereich außen mit einer Umfangsrille (29) zur Verstärkung des Sitzes im Befestigungsabschnitt (10) geformt ist.

5. Spritzeneinrichtung nach einem der Ansprüche 2 bis 4, **dadurch gekennzeichnet, dass** der gesonderte Kanülen-Anschlussteil (27) in seinem hinteren Bereich außen mit einem Umfangswulst (30) zur Verstärkung des Sitzes im Befestigungsabschnitt (10) geformt ist.

6. Spritzeneinrichtung nach einem der Ansprüche 2 bis 5, **dadurch gekennzeichnet, dass** im Verschlussabschnitt (11) ein radialer Absatz (22) geformt ist, der die Position des gesonderten Kanülen-Anschlussteils (27) nach vorne begrenzt.

7. Spritzeneinrichtung nach einem der Ansprüche 2 bis 6, **dadurch gekennzeichnet, dass** der gesonderte Kanülen-Anschlussteil (27) aus einem im Vergleich zum Material des Befestigungs- (10) und Verschlussabschnitts (11) harten Material, z.B. Polycarbonat, besteht.

8. Spritzeneinrichtung nach einem der Ansprüche 2 bis 7, **dadurch gekennzeichnet, dass** der Befestigungsabschnitt (10) für einen Sitz auf einem konischen Anschlussteil (6) des Spritzenkörpers (1) mit einem Innenkonus (6') ausgebildet ist.

9. Spritzeneinrichtung nach Anspruch 1, **dadurch gekennzeichnet, dass** der Befestigungsabschnitt (10') der Verschlusskappe (9') ringförmig ausgebildet ist.

10. Spritzeneinrichtung nach Anspruch 9, **dadurch gekennzeichnet, dass** der Kanülen-Anschlussteil (6) durch einen vorderen Anschluss-Abschnitt des Spritzenkörpers (1) gebildet ist, an dessen hinterem Ende der ringförmige Befestigungsabschnitt (10') gehalten ist.

11. Spritzeneinrichtung nach einem der Ansprüche 1 bis 10, **dadurch gekennzeichnet, dass** der Stopfen (24) unter axialer Kompression zwischen der vorderen Stirnseite des Kanülen-Anschlussteils (6;27) und der radialen Schulter (39) im Verschlussabschnitt (11) gehalten ist.

12. Spritzeneinrichtung nach einem der Ansprüche 1 bis 11, **dadurch gekennzeichnet, dass** der Stopfen (24) aus Latex-freiem Gummi besteht.

13. Spritzeneinrichtung nach einem der Ansprüche 1 bis 12, **dadurch gekennzeichnet, dass** der Verschlussabschnitt (11) an seiner Außenseite mit Griffvorsprüngen, z.B. Längsrippen (17), ausgebildet ist.

14. Spritzeneinrichtung nach Anspruch 1 bis 13, **dadurch gekennzeichnet, dass** der Befestigungsabschnitt (10) an seiner Außenseite mit Griffvorsprüngen, z.B. Längsrippen (16), ausgebildet ist.

15. Spritzeneinrichtung nach einem der Ansprüche 1 bis 14, **dadurch gekennzeichnet, dass** der Befestigungsabschnitt (10,10') mit Schnappverbindungsteilen (14, 15) für einen Schnappsitz auf einem Kanülen-Anschlussteil (6) des Spritzenkörpers (1) geformt ist.

16. Spritzeneinrichtung nach einem der Ansprüche 1 bis 15, **dadurch gekennzeichnet, dass** der Dichtkörper-Aufnahmeraum (23) im Verschlussabschnitt (11) mit Längsstegen (25) und dazwischen liegenden Lüftungskanälen (26) ausgebildet ist.

17. Spritzeneinrichtung nach einem der Ansprüche 1 bis 16, **dadurch gekennzeichnet, dass** der Befestigungsabschnitt (10,10') und der Verschlussabschnitt (11) ein Spritzgussteil aus Polyethylen sind.

18. Spritzeneinrichtung nach einem der Ansprüche 1 bis 17, **dadurch gekennzeichnet, dass** der Spritzenkörper (1) mit einer Injektionsflüssigkeit gefüllt und an seinem von der Verschlusskappe (9,9') abgewandten hinteren Ende mit einem Kolben (3) verschlossen ist.

19. Verschlusskappen-Einheit enthaltend einen länglichen Kanülen-Anschlussteil (27) einer Spritzeneinrichtung, einen den Kanülen-Anschlussteil abdichtenden Dichtkörper (24) und eine Verschlusskappe (9, 9') zum dichten Verschließen des Kanülen-Anschlussteils (27) vor der Anbringung der Kanüleneinheit (28), wobei die Verschlusskappe (9) zwei über eine Sollbruchstelle (12) einteilig miteinander verbundene Kappenabschnitte (10, 11) aufweist, von denen der eine ein über dem Kanülen-Anschlussteil (27) angebrachter hinterer, länglicher, zur Anbringung an einem Spritzenkörper (1) vorgesehener Befestigungsabschnitt (10) und der andere ein einen Aufnahmeraum (23) für den Dichtkörper (24) aufweisender vorderer Verschlussabschnitt (11) ist, wobei der mit einer axialen Bohrung versehene Kanülen-Anschlussteil (9) im Übergangsbereich von Befestigungsabschnitt (10) und Verschlussabschnitt (11), beidseits der Sollbruchstelle (12), angeordnet ist; **dadurch gekennzeichnet, dass** der Dichtkörper (24) ein zylindrischer Stopfen (24) ist, der zwischen der vorderen Stirnseite des Kanülen-Anschlussteils (6; 27) und einer den Aufnahmeraum (23) vorderseitig begrenzenden radialen Schulter (39) im Inneren des länglichen Verschlussabschnitts (11) der Verschlusskappe (9) eingelegt ist, und dass der Kanülen-Anschlussteil (9) als gesonderter Körper ausgebildet ist, der einen Sitz sowohl im Befestigungsabschnitt (10) als auch im Verschlussabschnitt (11) der Verschlusskappe (9) aufweist, wobei er im Befestigungsabschnitt (10) einen festeren Sitz als im Verschlussabschnitt (11) aufweist.

20. Verschlusskappen-Einheit nach Anspruch 19, **dadurch gekennzeichnet, dass** der Kanülenaufnahmebereich des Kanülen-Anschlussteils (27) als Luer-Konus (27') ausgeführt ist.

21. Verschlusskappen-Einheit nach Anspruch 19 oder 20, **dadurch gekennzeichnet, dass** im hinteren Bereich des Kanülen-Anschlussteils (27) außen eine Umfangsrille (29) geformt ist.

22. Verschlusskappen-Einheit nach einem der Ansprüche 19 bis 21, **dadurch gekennzeichnet, dass** im hinteren Bereich des Kanülen-Anschlussteils (27) außen ein Umfangswulst (30) geformt ist.

23. Verschlusskappen-Einheit nach einem der Ansprüche 19 bis 22, **dadurch gekennzeichnet, dass** der Kanülen-Anschlussteil (27) aus Polycarbonat besteht.

## Claims

1. A syringe device comprising a syringe body (1) provided with a cannula connecting part (6; 27) with an axial bore (33, 34) for attaching a cannula unit (28) thereon at its one, front end, a sealing body (24) sealing the cannula connecting part, and a closure cap (9, 9') sealingly closing the cannula connecting part prior to application of the cannula unit (28), the closure cap including two cap portions (10, 11) integrally interconnected via a pre-determined breaking point (12), one of the cap portions being a rear fastening portion (10, 10') attached to the cannula connecting part, and the other one being a front closure portion (11) including a receiving space (23) for the sealing body (24), **characterised in that** the sealing body (24) is a cylindrical plug (24) inserted between the front end side of the cannula connecting part (6; 27) and a radial shoulder (39) delimiting the receiving space (23) at its front side in the interior of the elongate closure portion (11) of the closure cap (9, 9').

2. A syringe device according to claim 1, **characterised in that** at the transition between the fastening portion (10) and the closure portion (11), on either side of the pre-determined breaking point (12), inwardly a separate cannula connecting part (27) provided with an axial bore is accommodated which is seated in the elongate fastening portion (10) more tightly than in the closure portion (11).

3. A syringe device according to claim 2, **characterised in that** the front region of the separate cannula connecting part (27) that is located in the closure portion (11) is designed as a Luer cone.

4. A syringe device according to claim 2 or 3, **characterised in that** the separate cannula connecting part (27) in its rear region is shaped with an outer peripheral groove (29) so as to strengthen the seat in the fastening portion (10).

5. A syringe device according to any one of claims 2 to 4, **characterised in that** the separate cannula connecting part (27) in its rear region is shaped with an outer peripheral bead (30) for strengthening the seat in the fastening portion (10).

6. A syringe device according to any one of claims 2 to 5, **characterised in that** a radial shoulder (22) is formed in the closure portion (11) which delimits the position of the separate cannula connecting part (27) in forward direction.

7. A syringe device according to any one of claims 2 to 6, **characterised in that** the separate cannula connecting part (27) is made of a material that is hard as compared to the material of the fastening (10) and closure portion (11), e.g. of polycarbonate.

8. A syringe device according to any one of claims 2 to 7, **characterised in that** the fastening portion (10) is designed with an inner cone (6') for a seat on a conical connecting part (6) of the syringe body (1).

9. A syringe device according to claim 1, **characterised in that** the fastening portion (10') of the closure cap (9') is designed to be annular.

10. A syringe device according to claim 9, **characterised in that** the cannula connecting part (6) is comprised by a front: connecting portion of the syringe body (1), at whose rearward end the annular fastening portion (10') is held.

11. A syringe device according to any one of claims 1 to 10, **characterised in that** the plug. (24) is held axially compressed between the front end side of the cannula connecting part (6; 27) and the radial shoulder (39) in the connecting portion (11).

12. A syringe device according to any one of claims 1 to 11, **characterised in that** the plug (24) is made of latex-free rubber.

13. A syringe device according to any one of claims 1 to 12, **characterised in that** the closure portion (11) is provided with gripping projections, e.g. longitudinal ribs (17), at its outer side.

14. A syringe device according to claim 1 to 13, **characterised in that** the fastening portion (10) is provided with gripping projections, e.g. longitudinal ribs (16), at its outer side.

15. A syringe device according to any one of claims 1 to 14, **characterised in that** the fastening portion (10, 10') is formed with snap connection means (14, 15) for a snap seat on a cannula connecting part (6) of the syringe body (1).

16. A syringe device according to any one of claims 1 to 15, **characterised in that** the sealing body receiving space (23) in the closure portion (11) is formed with longitudinal webs (25) and venting channels (26) arranged therebetween.

17. A syringe device according to any one of claims 1 to 16, **characterised in that** the fastening portion (10, 10') and the closure portion (11) are an injection-molded part of polyethylene.

18. A syringe device according to any one of claims 1 to 17, **characterised in that** the syringe body (1) is filled with an injection liquid and closed at its rear end that faces away from the closure cap (9, 9') by a piston (3).

19. A closure cap unit comprising an elongate cannula connecting part (27) of a syringe device, a sealing body (24) sealing the cannula connecting part, and a closure cap (9, 9') for sealingly closing the cannula connecting part (27) prior to attachment of the cannula unit (28), the closure cap (9) having two cap portions (10, 11) integrally interconnected via a pre-determined breaking point (12), one of which being a rearward, elongate fastening portion (10) mounted on the cannula connecting part (27) and provided to be attached to a syringe body (1), and the other one being a front closure portion (11) having a receiving space (23) for the sealing body (24), the cannula connecting part (27) having an axial bore and being arranged in the transition region of the fastening portion (10) and the closure portion (11) on either side of the pre-determined breaking point (12), **characterised in that** the sealing body (24) is a cylindrical plug (24) inserted between the front end side of the cannula connecting part (6; 27) and a radial shoulder (39) delimiting the receiving space (23) on its front side, in the interior of the elongate closure portion (11) of the closure cap (9), and that the cannula connecting part (27) is designed as a separate body which is seated both in the fastening portion (10) and in the closure portion (11) of the closure cap (9), it being seated more tightly in the fastening portion (10) than in the closure portion (11).

20. A closure cap unit according to claim 19, **characterised in that** the cannula receiving area of the cannula connecting part (27) is designed as a Luer cone (27').

21. A closure cap unit according to claim 19 or 20, **characterised in that** in the rear region of the cannula connecting part (27), an external peripheral groove (29) is shaped.

22. A closure cap unit according to any one of claims 19 to 21, **characterised in that** in the rear region of the cannula connecting part (27), externally a peripheral bead (30) is formed.

23. A closure cap unit according to any one of claims 19 to 22, **characterised in that** the cannular connecting part (27) is made of polycarbonate.

## Revendications

1. Dispositif d'injection comprenant un corps d'injection (1), sur l'extrémité avant duquel est prévue une pièce de raccordement de canule (6 ; 27) ayant un alésage axial (33, 34) pour le montage d'une unité de canule (28), comprenant un élément d'étanchéité (24) qui rend étanche la pièce de raccordement de canule et un bouchon de fermeture (9, 9') pour fermer de façon étanche la pièce de raccordement de canule avant le montage de l'unité de canule (28), où le bouchon de fermeture présente deux parties de bouchon (10, 11) reliées entre elles d'une seule pièce par un point destiné à la rupture (12), dont l'une est une partie de fixation arrière (10, 10') agencée sur la pièce de raccordement de canule et l'autre est une partie de fermeture avant (11) comprenant un espace de logement (23) pour l'élément d'étanchéité (24), **caractérisé en ce que** l'élément d'étanchéité (24) est un bouchon cylindrique (24) qui est inséré entre le côté d'extrémité avant de la pièce de raccordement de canule (6 ; 27) et un épaulement radial (39) délimitant l'espace de logement (23) du côté avant à l'intérieur de la partie de fermeture longitudinale (11) du bouchon de fermeture (9, 9').

2. Dispositif d'injection selon la revendication 1, **caractérisé en ce que**, dans la zone de transition de la partie de fixation (10) et de la partie de fermeture (11), sur les deux côtés du point destiné à la rupture (12), une pièce de raccordement de canule (27) séparée, munie d'un alésage axial est prévue à l'intérieur, dont le siège est plus stable dans la partie de fixation longitudinale (10) que dans la partie de fermeture (11).

3. Dispositif d'injection selon la revendication 2, **caractérisé en ce** la pièce de raccordement de canule séparée (27) est réalisée avec sa zone avant située dans la partie de fermeture (11) comme cône Luer.

4. Dispositif d'injection selon l'une quelconque des revendications 2 ou 3, **caractérisé en ce que** la pièce de raccordement de canule séparée (27) est formée dans sa zone arrière, à l'extérieur, avec une rainure périphérique (29) pour renforcer le siège dans la partie de fixation (10).

5. Dispositif d'injection selon l'une quelconque des revendications 2 à 4, **caractérisé en ce que** la pièce de raccordement de canule séparée (27) est formée dans sa zone arrière, à l'extérieur, avec un renflement périphérique (30) pour renforcer le siège dans la partie de fixation (10).

6. Dispositif d'injection selon l'une quelconque des revendications 2 à 5, **caractérisé en ce qu'**un palier radial (22) qui limite la position de la pièce de raccordement de canule séparée (27) vers l'avant est formé dans la partie de fermeture (11).

7. Dispositif d'injection selon l'une quelconque des revendications 2 à 6, **caractérisé en ce que** la pièce de raccordement de canule séparée (27) est constituée d'un matériau dur, par exemple de polycarbonate, par rapport au matériau des parties de fixation (10) et de fermeture (11).

8. Dispositif d'injection selon l'une quelconque des revendications 2 à 7, **caractérisé en ce que** la partie de fixation (10) est réalisée avec un cône intérieur (6') pour former un siège sur une pièce de raccordement conique (6) du corps d'injection (1).

9. Dispositif d'injection selon la revendication 1, **caractérisé en ce que** la partie de fixation (10') du bouchon de fermeture (9') est réalisée dans une forme annulaire.

10. Dispositif d'injection selon la revendication 9, **caractérisé en ce que** la pièce de raccordement de canule (6) est réalisée par une partie de raccordement avant du corps d'injection (1), à l'extrémité arrière de laquelle la partie de fixation annulaire (10') est maintenue.

11. Dispositif d'injection selon l'une quelconque des revendications 1 à 10, **caractérisé en ce que** le bouchon (24) est maintenu sous une compression axiale entre le côté d'extrémité avant de la pièce de raccordement de canule (6 ; 27) et l'épaulement radial (39) dans la partie de fermeture (11).

12. Dispositif d'injection selon l'une quelconque des revendications 1 à 11, **caractérisé en ce que** le bouchon (24) est constitué de caoutchouc sans latex.

13. Dispositif d'injection selon l'une quelconque des revendications 1 à 12, **caractérisé en ce que** la partie de fermeture (11) est réalisée sur son côté extérieur avec des saillies de prise, par exemple des nervures longitudinales (17).

14. Dispositif d'injection selon l'une quelconque des revendications 1 à 13, **caractérisé en ce que** la partie de fixation (10) est réalisée sur son côté extérieur avec des saillies de prise, par exemple des nervures longitudinales (16).

15. Dispositif d'injection selon l'une quelconque des revendications 1 à 14, **caractérisé en ce que** la partie de fixation (10) est formée avec des pièces d'assemblage à enclenchement (14, 15) pour un siège à enclenchement sur une partie de raccordement de canule (6) du corps d'injection (1).

16. Dispositif d'injection selon l'une quelconque des revendications 1 à 15, **caractérisé en ce que** l'espace de logement (23) de l'élément d'étanchéité est réalisé dans la partie de fermeture (11) avec des arêtes longitudinales (25) et des canaux d'aération (26) situées entre celles-ci.

17. Dispositif d'injection selon l'une quelconque des revendications 1 à 16, **caractérisé en ce que** la partie de fixation (10, 10') et la partie de fermeture (11) sont une pièce moulée par injection en polyéthylène.

18. Dispositif d'injection selon l'une quelconque des revendications 1 à 17, **caractérisé en ce que** le corps d'injection (1) est rempli d'un liquide d'injection et fermé à son extrémité arrière opposée au bouchon de fermeture (9, 9') par un piston (3).

19. Unité de bouchon de fermeture comprenant une pièce de raccordement de canule longitudinale (27) d'un dispositif d'injection, un élément d'étanchéité (24) qui rend étanche la pièce de raccordement de canule et un bouchon de fermeture (9, 9') pour fermer de façon étanche la pièce de raccordement de canule (27) avant le montage de l'unité de canule (28), où le bouchon de fermeture (9) présente deux parties de bouchon (10, 11) reliées entre elles d'une seule pièce par un point destiné à la rupture (12), dont l'une est une partie de fixation (10) arrière, longitudinale, prévue pour le montage sur un corps d'injection (1), agencée au moyen de la pièce de raccordement de canule (27), et l'autre est une partie de fermeture avant (11) comprenant un espace de logement (23) pour l'élément d'étanchéité (24), où la pièce de raccordement de canule (9) munie d'un alésage axial est agencée dans la zone de transition de la partie de fixation (10) et de la partie de fermeture (11), sur les deux côtés du point destiné à la rupture (12), **caractérisée en ce que** l'élément d'étanchéité (24) est un bouchon cylindrique (24) qui est inséré entre le côté d'extrémité avant de la pièce de raccordement de canule (6 ; 27) et un épaulement radial (39) délimitant l'espace de logement (23) du côté avant à l'intérieur de la partie de fermeture longitudinale (11) du bouchon de fermeture (9), et **en ce que** la pièce de raccordement de canule (9) est réalisée comme élément séparé qui présente un siège aussi bien dans la partie de fixation (10) que dans la partie de fermeture (11) du bouchon de fermeture (9), ayant un siège plus stable dans la partie de fixation (10) que dans la partie de fermeture (11).

20. Unité de bouchon de fermeture selon la revendication 19, **caractérisée en ce que** la zone de logement de canule de la pièce de raccordement de canule (27) est réalisée comme cône Luer (27').

21. Unité de bouchon de fermeture selon l'une quelconque des revendications 19 ou 20, **caractérisée en ce qu'**une rainure périphérique (29) est formée dans la zone arrière à l'extérieur de la pièce de raccordement de canule (27).

22. Unité de bouchon de fermeture selon l'une quelconque des revendications 19 à 21, **caractérisée en ce qu'**un renflement périphérique (30) est formé dans la zone arrière à l'extérieur de la pièce de raccordement de canule (27).

23. Unité de bouchon de fermeture selon l'une quelconque des revendications 19 à 22, **caractérisée en ce que** la pièce de raccordement de canule (27) est constituée de polycarbonate.
